# EUROPEAN PATENT APPLICATION

(11) **EP 3 469 980 A1**
(43) Date of publication of application: **17.04.2019**
(21) Application number: 17195583.4
(22) Date of filing: 10.10.2017
(51) Int. Cl.: A61B 5/00, A61B 5/107

(54) **A MONITORING DEVICE FOR MONITORING BREAST MILK CONSUMPTION**

(71) Applicant: Koninklijke Philips N.V., 5656 AE Eindhoven (NL)
(72) Inventor: TIAN, Cong, 5656 AE Eindhoven (NL)
(74) Representative: de Haan, Poul Erik

(57) **Abstract**

A monitoring device is for monitoring breast milk consumption during breastfeeding in which breast tissue stiffness changes are monitored in order to determine breast milk consumption information.

## Description

### FIELD OF THE INVENTION

This invention relates to devices for monitoring breast milk consumption during feeding.

### BACKGROUND OF THE INVENTION

Breastfeeding mothers are very interested to know how much milk their baby has consumed from each breast, in order to track the feeding times and milk quantities consumed by the baby with the growth of the baby. Of interest for example are the date, time and side of breastfeeding, the average amount of milk consumed per feed session, the breast productivity at different times of the day, and the weight gain of the baby over time.

One approach is to monitor changes in the mammary alveoli in order to determine the amount of milk the infant receives from the breast. For example, a monitoring product known as "Milksense" (trade mark) traces changes in capacitance and resistance of the breast tissue before and after breastfeeding. The monitor transmits 40 kHz and 20 kHz signals to the breast tissue at an electric current of about 0.5mA and measures the response signals via electrodes in contact with the skin. The measurement is sensitive to the average volume of mammary alveoli in the breast.

However, the bio-impedance technology used in this approach may change with multiple factors, such as fat mass content, level of edema and breathing cycle. In this case, the pattern of the bio-impedance signals needs to be identified beforehand, and then is used to indicate the volume of breastfeed milk. However, this does not give very precise measurements. There is also a concern expressed by mothers relating to the electrical current passing through their breast tissue and concerns over whether this could have an impact on the breastmilk and on their breast shape.

US 2012/0277636 discloses an alternative approach based on volume monitoring. An inflatable bag is placed over the breast. The volume of added air to the bag corresponds to the reduction in breast volume and is mapped to the amount of milk produced.

This approach can only be effective for a large quantity of breast milk. Since as little as 20 ml may be extracted from one breast, this volume may represent only the fluid volume in the breast duct whereas the volume change of the breast, which mainly depends on the breast fat tissue, may not be detectable. Furthermore, the movement caused by pumping or breastfeeding can interfere with the measurement of a change of breast volume.

There remains a need for a monitor which is electrically passive, but which enables an accurate determination of the breastmilk consumption and/or production.

### SUMMARY OF THE INVENTION

It is a concept of the invention to make use of stiffness measurements to determine levels of breast milk consumption.

The invention is defined by the claims.

According to examples in accordance with an aspect of the invention, there is provided a monitoring device for monitoring breast milk consumption during breastfeeding, comprising:
a breast stiffness monitoring unit; and
a controller,
wherein the controller is adapted to monitor stiffness changes resulting from breast milk expression thereby to determine the breast milk consumption.

This device is able to monitor milk consumption by monitoring breast stiffness. This can be achieved without using electrical stimulation signals, as are for example known for tissue analysis. It is also more accurate than measurement of changes in breast volume and can also obtain measurements over a longer period of time and for smaller volume production amounts.

The device may be incorporated into a breast shield or breast massaging device or a breast pump. It can simply be worn by the mother and provides a non-invasive monitoring function. Knowledge of the infant's consumption of breast milk is of interest both for monitoring the development of the baby and for the wellbeing of the mother. The same device may also be used for general breast monitoring, for example for examining a breast lump.

The breast stiffness monitoring unit may comprise a deformation sensor, and a pressure or force applicator. There may also be a force or pressure sensor for providing feedback about the applied force or pressure.

These together allow stiffness monitoring. A pressure or force is applied, and the reaction to this pressure or force (as a deformation) is measured.

The pressure or force applicator may comprise one or more inflatable bags for applying pressure to the breast. This provides a simple and non-invasive way to apply pressure. A pressure sensor may then be provided for sensing the pressure applied towards the breast by the one or more inflatable bags. The stiffness of the breast can be calculated based on the pressure applied towards the breast and resulting deformation.

An alternative example of force or pressure applicator is a suction system for applying suction pressure. This may be the suction of a breast pump. This provides a simple and non-invasive way to apply negative pressure. A pressure sensor may then be provided for sensing the negative pressure in the suction hole of breast pump. The suction pressure then induces the deformation of the breast. The degree of deformation of the breast can be measured by the deformation sensor.

In both cases, the stiffness of the breast can be calculated based on the pressure (negative or positive) applied and the resulting deformation.

In both cases, there may be a deformation sensor having an array of deformation sensor elements. As a minimum, one deformation sensor element may suffice to provide a stiffness indication. A single deformation sensor element may for example be moved around the breast to obtain multiple measurements. However, an array may instead be used, for example if the sensor is to have a fixed configuration, such as incorporated into a breast shield or breast pump.

The use of multiple sensor elements enables the stiffness characteristic to be monitored over an area of the breast, in simultaneous manner. A stiffness distribution map may in this way be obtained. This may identify local regions of high stiffness such as caused by a lump or milk plug (e.g. caused by a blocked milk duct giving rise to mastitis). These may be screened out from the analysis so that they do not affect the breast milk consumption data, and they may also be used to provide separate advisory information, such as a warning indicator to help avoiding mastitis.

The monitoring unit may comprise a ring for placement around the nipple. Preferably, the ring is for placement around the outside of the areola, and it may for example comprise part of a nipple shield or part of a breast pump.

The controller may be further adapted to identify a milk plug or bulge. As mentioned above, this may be used to provide more accurate monitoring by filtering collected data or it provide additional information, for example advising the mother to carry out a self-examination for avoiding mastitis. The device may be used as a breast monitor even after the lactation period.

The device may form part of a breast pump. Before and after breastfeeding, the breast pump may be applied to evaluate how much breast milk was consumed by the baby between those times. The mother may then also use the pump after breastfeeding for emptying the breast and maintaining breastfeeding capacity. In this example, the stiffness before breastfeeding indicates the baseline of breastmilk, and the stiffness after breastfeeding indicates the remaining breastmilk and the difference represents the milk consumed by the baby.

By incorporating the device into a breast pump, the number of products needed by the mother is reduced.

The controller may be adapted to implement a calibration routine based on input from a user indicating an amount of breastmilk obtained from using the breast pump.

The breast pump may in this way also be used to calibrate the function which derives the quantity of breastmilk consumed from the stiffness information. During a breast pump expression, the user may provide as input the known volume of breastmilk, either manually or as a readout from a smart milk bottle with a scale. The device can then calculate the stiffness information automatically and linear regression or other algorithms can be used to calibrate the conversion between stiffness and milk volume for that particular user.

The invention also provides a method of monitoring breast milk consumption during breastfeeding, comprising:
monitoring breast stiffness changes resulting from breast milk expression thereby to determine the breast milk consumption.

The method may comprise applying pressure using a pressure applicator and sensing a resulting deformation or pressure. Applying pressure may comprise inflating one or more inflatable bags. In another case, applying pressure may comprise applying suction pressure induced by a breast pump. The method may further comprise identifying a breast plug from the monitored breast stiffness.

The method may comprise calibrating the determining of the breast milk consumption using milk production information from the use of a breast pump.

The invention may be implemented, at least in part, in software.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Fig. 1 shows an image of a breast which is full of milk (left image) and after emptying (right image);
Fig. 2 shows a monitoring device for monitoring breast milk consumption during breastfeeding;.
Fig. 3 shows a method of monitoring breast milk consumption during breastfeeding; and
Fig. 4 shows test results which demonstrate the feasibility of the approach.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a monitoring device for monitoring breast milk consumption during breastfeeding in which breast tissue stiffness changes are monitored in order to determine breast milk consumption information. The breast stiffness may be monitored during natural breastfeeding, or it may be monitored before and after breastfeeding, for example using a monitoring device associated with a breast pump or nipple shield. The mechanical properties of the breast, in particular the stiffness, provide an indicator of breast fullness which, based on change information, can be used to estimate the breast milk consumption by a baby.

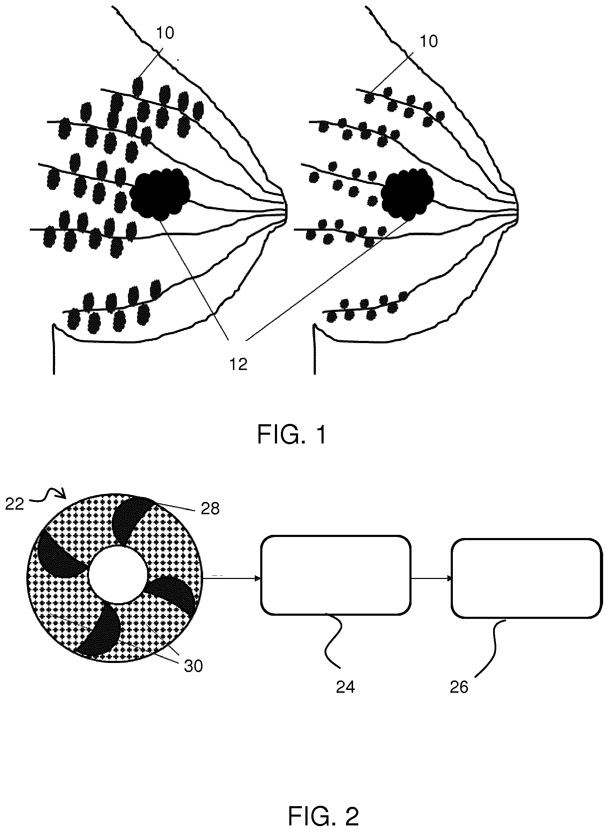

Fig. 1 shows an image of a breast which is full of milk (left image) and after emptying (right image).

The breast contains mammary alveoli 10 which have increased size when the breast is full. After emptying the breast, the stiffness will decrease as a result of the reduced size of the alveoli as well as less breast milk in the alveoli 10. The area 12 indicates a breast plug. A local high stiffness measurement may also be used to identify the milk plug 12 and also screen out its influence on the measurements.

Fig. 2 shows a monitoring device 20 for monitoring breast milk consumption during breastfeeding. The device comprises a breast stiffness monitoring unit 22, a controller 24 and an output interface 26 such as a display. The controller monitors stiffness changes over a time of breast milk expression thereby to determine the breast milk consumption.

The measurement of a stiffness avoids the need for electrical stimulation and provides accurate measurement results even for small changes in breast milk consumption (or production).

The monitoring unit 22 generally comprises a ring for placement around the nipple, for example around the outside of the areola, and forms a breast shaped patch. It may for example comprise part of a nipple shield or part of a breast pump.

The monitoring unit in this example comprises a set of inflatable air bags 28 (although there may be only one), and one or multiple deformation sensors 30 to monitor the deformation induced by compression caused by the one or more inflatable air bags 28. There may also be force or pressure sensors for providing feedback in respect of the applied pressure or force, i.e. the pressure applied by the inflatable air bags in this example. In the example shown, the deformation sensors 30 have a fixed position and the user does not need make any position adjustment.

The pressure applied by the air bags may be estimated based on the volume change of the air in the air bags, or it may be measured by means of an air pressure gauge. The pressure level and duration is controlled by the main control unit 24 within a predetermined safety range.

The collected data is then used for calculating a map of stiffness and also a mean value of stiffness for the breast as a whole.

The main control unit 24 may be embodied in the patch or it may embodied in another separate device communicating with the patch via wired or wireless methods. It calculates a map of stiffness and determines the information relating to breastmilk consumption and also identification of a breast plug. This information is provided as output to the output interface 26, i.e. the display.

A display may be part of an external device such as a portable device with which the monitoring device communicates (a mobile phone, computer or tablet), or it may be a display which forms part of the monitoring device such as a flexible display upon the breast.

The deformation sensors for example comprise strain gauges or optical sensors which are embodied in the patch.

The stiffness information for the body of the breast (outside of the areola) may be derived by using an equation which relates its deformation to the pressure applied to the breast. However, there may instead be a direct conversion from the deformation and pressure information to the breast fullness measure without any need for a determination of an actual stiffness value. Thus more generally, the invention provides conversion between force and deformation (which together are indicative of stiffness) and a breast milk quantity.

When a stiffness value is determined, the monitoring device may determine a single average stiffness value but more preferably a map of stiffness values is obtained giving a breast stiffness profile. This profile can also be used to screen out a milk plug or a bulge in the breast lobe from the calculations. The device can then also be used to generate an advisory message that a self-examination of the breast is desirable.

The stiffness information is then mapped to a degree of fullness of the breast. By monitoring a change of mean stiffness of the breast (outside the area of the areola) during (or before and after) breast feeding, the amount of milk expressed can be determined.

An average stiffness is of interest for determining the breast fullness. However, the stiffness readings from different areas of the breast may be combined in more complicated ways so that a simple average is not the only way to obtain a single representative parameter. By analyzing the stiffness values individually as well as forming an average (or other single representative value), a distribution map may be obtained and local regions of high stiffness such as caused by a lump or milk plug can be identified. These local extreme values may be removed from the calculation of the average (or other single representative value).

As mentioned above, the device may form part of a breast pump. Before and after breastfeeding, the breast pump may be applied to evaluate how much breast milk was consumed by the baby during breastfeeding between those times. The mother may then also use the breast pump after breastfeeding for emptying the breast. This assists in maintaining breastfeeding capacity.

The pressure applicator may also be based on the suction pump of the breast pump. This provides an alternative example of force or pressure applicator. When a negative pressure is applied to the nipple by a breast pump, there is again a deformation of the breast, which will depend on the stiffness of the breast. A pressure sensor may then be provided for sensing the negative pressure in the suction hole of the breast pump to provide a measure of the pressure applied which is then combined with the measured deformation to derive the stiffness measure. The stiffness measure may thus either be based on a direct measure of the deformation of breast tissue resulting from application of force to the same (or very close) breast tissue as in the air bag example, or it may be based on the more indirect breast tissue deformation resulting from application of suction by the breast pump. Both examples give a measure of stiffness. The same deformation sensor may be used for the breast pump example as for the air bag example described above. A calibration routine can again provide the relationship between the negative pressure applied by the breast pump and the resulting deformation of the breast, for example within the volume of the breast pump cavity.

The change in stiffness observed before and after breastfeeding is then converted to a change in breast fullness. Thus, in this case, the monitoring does not take place during breastfeeding itself. Instead, the monitoring device may be applied during breastfeeding itself (for example as part of a nipple shield worn during breast feeding) and this will enable stiffness monitoring over time during the breastfeeding.

The relationship between breast stiffness changes and breast fullness may vary between different individuals. These differences can be tolerated by using a calibration routine. This basically involves expressing known amounts of milk and monitoring the breast stiffness (or more generally the force applied and resulting deformation).

One way to obtain this calibration information is to monitor breast stiffness during milk extraction using a breast pump, when the milk volume produced can be directly measured. The change is stiffness over time can then be mapped to the change in breast fullness over time. The user may enter a volume of milk produced after expression, or even at multiple times during expression. Alternatively, a smart milk collection vessel may provide this information continuously in automated manner.

A breast pump thus provides calibration of the function which relates stiffness to breast fullness.

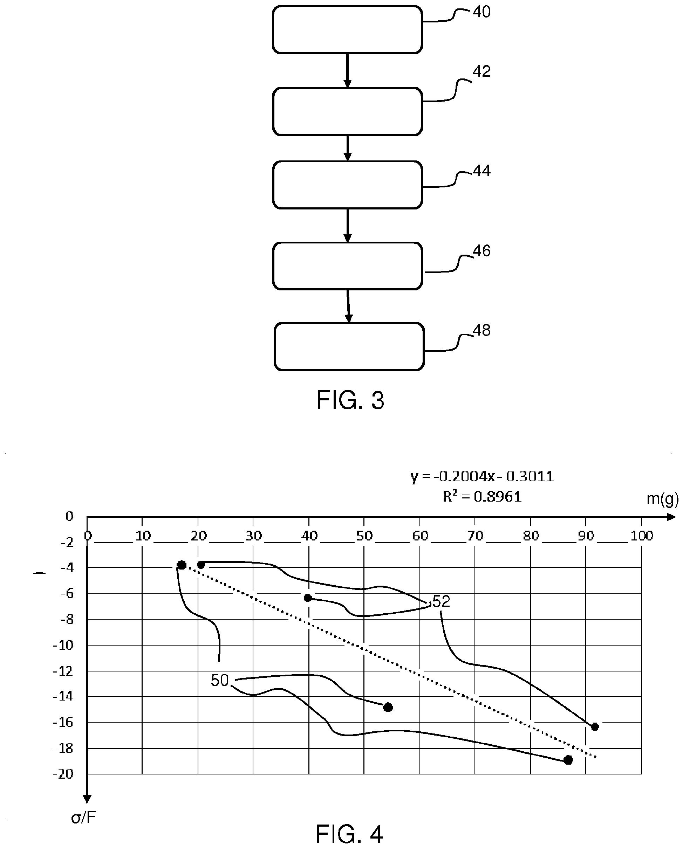

Fig. 3 shows a method of monitoring breast milk consumption during breastfeeding.

A calibration routine comprises expressing milk using a breast pump in step 40, and monitoring the breast stiffness (in particular an average breast stiffness, including optionally a signal filtering function) in step 42. This may be involve reporting the amount of milk expressed either at the end of the breast pump expression or at multiple times during the expression. The breast stiffness may be based on a direct measure of deformation in response to force applied to the breast tissue (as with the air bag example) or it may be based on the indirect deformation of the breast resulting from the negative pressure applied by a breast pump.

In step 44, a relationship is derived between the breast fullness and the stiffness for that particular mother.

In step 46 the breast stiffness is monitored either before and after breastfeeding, or during breastfeeding.

In step 48, the corresponding breast milk consumption is derived using the relationship obtained in step 44.

Of course, the calibration only needs to take place once for the particular mother.

The feasibility of using breast stiffness for determining breast fullness has been tested, using a commercially available pressure sensor and strain sensor. The sensor arrangement was pressed manually against the breast (instead of using an air bag arrangement) to obtain stiffness measurements.

A peak value of applied pressure and measured deformation during a measurement was used to calculate the stiffness of breast in a breast full status. The breast stiffness was measured in the same way at the middle of a breast milk expression and the breast milk produced was measured using a scale. The breast stiffness was also measured at the end of the breast milk expression in the same way and the breast milk produced was measured. Thus, three different measurement were taken for one expression cycle.

This process was then repeated in different days.

Fig. 4 shows the results for two days, and plots the milk production (in grams) versus the stiffness indicator (no units). Points 50 are for one day and points 52 are for another day.

The data shows a strong correlation between the stiffness (an index of strain/force* 100) and the mass of breast milk produced (with a linear regression value of R²=0.9, p<0.05).

The tests performed have thus shown a strong correlation between the breast stiffness and the breast milk expression (i.e. consumption in the case of breastfeeding). Data from multiple days also shows repeatability over time.

In the test example above, a strain gauge was used to measure deformation directly. The deformation measurement could comprise a sensor for detecting flexing or a light sensor for detecting distance. In the example above, a piezoelectric sensor was used to measure the pressure applied towards the breast (representing the inflating airbags).

The invention may make use of any suitable measure of stiffness, i.e. any value representing the resistance offered by the breast to deformation. The stiffness may be measured along one direction only, preferably normally to the skin surface and inwardly at the point of measurement, and it may then simply comprise a ratio of the force applied to the skin to the displacement (i.e. the change in position compared to position at which zero external force is applied). This displacement is associated with a strain value, and the stiffness is associated with a Young's modulus, but an actual strain value and an actual Young's modulus value do not need to be determined. For example there is no need for determination of a nominal total length of the tissue being deformed.

The use of a calibration procedure in particular enables any suitable force and deformation measurements (or associated stiffness measurement) to be mapped to a corresponding breast fullness measure. A best fit approach then enables a function to be derived which relates the two values. This function may be linear or non-linear.

The example above has an array of deformation sensor elements. There may be only one deformation sensor element, which may be moved around the breast manually to obtain an average stiffness level.

In the example above, there is a calculation of a stiffness value and then conversion to a breast fullness measure. The stiffness is thus an intermediate variable and as such it does not need to be calculated or output; in practice the controller receives as inputs the pressure applied and the deformation measured and outputs the breast fullness indication. Thus, the stiffness changes do not need to be measured or monitored directly but may be performed implicitly when converting from pressure and deformation values to the breast milk consumption level or breast fullness level.

The primary interest is for monitoring breast milk consumption during breast feeding. However, the device can clearly also be used for determining the evolution of the breast fullness level at times between breastfeeding.

The data processing operations carried out the controller 24 may be carried out in software. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A monitoring device for monitoring breast milk consumption during breastfeeding, comprising:
a breast stiffness monitoring unit (22) and
a controller (24),
wherein the controller is adapted to monitor stiffness changes resulting from breast milk expression thereby to determine the breast milk consumption.

2. A device as claimed in claim 1, wherein the breast stiffness monitoring unit (22) comprises a deformation sensor (30), and a force or pressure applicator (28).

3. A device as claimed in claim 2, comprising a deformation sensor having an array of deformation sensor elements (30).

4. A device as claimed in claim 2 or 3, wherein the force or pressure applicator comprises:
one or more inflatable bags (28); or
a suction pump.

5. A device as claimed in claim 4, further comprising a pressure sensor for sensing the pressure applied towards the breast by the one or more inflatable bags or the negative pressure applied by the suction pump.

6. A device as claimed in any preceding claim, wherein monitoring unit (22) comprises a ring for placement around the nipple.

7. A device as claimed in any preceding claim, wherein the controller (24) is further adapted to identify a milk plug or bulge.

8. A device as claimed in any preceding claim, forming part of a breast pump.

9. A device as claimed in claim 8, wherein the controller is adapted to implement a calibration routine based on input from a user indicating an amount of breastmilk obtained from using the breast pump.

10. A method of monitoring breast milk consumption during breastfeeding, comprising:
monitoring breast stiffness changes resulting from breast milk expression thereby to determine the breast milk consumption.

11. A method as claimed in claim 10, comprising applying pressure using a pressure applicator and sensing a resulting deformation or pressure.

12. A method as claimed in claim 11, wherein applying pressure comprises:
inflating one or more inflatable bags; or
applying suction pressure induced by a breast pump.

13. A method as claimed in any one of claims 10 to 12, further comprising identifying a breast plug from the monitored breast stiffness.

14. A method as claimed in any one of claims 10 to 13, comprising calibrating the determining of the breast milk consumption using milk production information from the use of a breast pump.

15. A computer program comprising computer program code means which is adapted, when said program is run on a computer, to implement the method of any one of claims 10 to 14.
